# EUROPEAN PATENT SPECIFICATION

(11) **EP 0 418 704 B1**
(45) Date of publication and mention of the grant of the patent: **15.12.1993**
(21) Application number: 90117442.5
(22) Date of filing: 11.09.1990
(51) Int. Cl.: A23J 1/20, C07K 15/00, C12N 9/08

(54) **Process of separating, purifying and recovering milk proteins capable of binding iron**
Verfahren zur Trennung, Reinigung und Gewinnung von eisenbindendem Milchprotein
Procédé de séparation, purification et récupération de protéines du lait capables de fixer du fer

(30) Priority: 22.09.1989 JP 247326/89
(43) Date of publication of application: 27.03.1991
(73) Proprietor: SNOW BRAND MILK PRODUCTS CO., LTD., Sapporo-shi, Hokkaido 065 (JP)
(72) Inventor: Sato, Kaoru, Arajukucho, Kawagoe-shi, Saitama-ken (JP); Dousako, Shun-ichi, Urawa-shi, Saitama-ken (JP)
(74) Representative: Boeters, Hans Dietrich, Dr.

(56) References cited:
- EP-A- 0 298 875
- US-A- 4 667 018
- PATENT ABSTRACTS OF JAPAN, vol. 13, no. 63 (C-568)[3411], 13th February 1989; & JP-A-63 255 299 (SNOW BRAND MILK PROD. CO., LTD) 21-10-1988
- PATENT ABSTRACTS OF JAPAN, vol. 13, no. 63 (C-568)[3411], 13th February 1989; & JP-A-63 255 300 (SNOW BRAND MILK PROD. CO., LTD) 21-10-1988

## Description

The present invention relates to the method of separating, purifying and recovering lactoperoxidase and lactoferrin or only lactoperoxidase from solutions that contain these proteins capable of binding iron.

### Description of the Prior Art

Lactoperoxidase and lactoferrin are proteins capable of binding iron, found in milk and other types of exocrine, and are both known to have bactericidal properties. Lactoperoxidase is an indispensable substance for labeling protein with iodine, while lactoferrin has such physiological functions as encouraging growth of lymphocytes, promoting absorption of iron, regulating differentiation of leukocytes, inhibiting lipid peroxidation and antiviral effect. Because of these properties, lactoperoxidase and lactoferrin can be useful as pharmaceuticals, and are important as feeds and food materials.

For some time, efforts have been made to separate lactoperoxidase and lactoferrin from milk, but it has been difficult to separate these two efficiently by one treatment, since lactoperoxidase and lactoferrin are both proteins capable of binding iron, and are similar in molecular weights and isoelectric points. For example, a method of separating lactoperoxidase and lactoferrin simultaneously by allowing milk to contact alginate gel (Japanese Patent Laid-Open Publication No. 61-246198), has been known. This method gives lactoperoxidase and lactoferrin in one fraction, but requires further purifying process to separate lactoperoxidase and lactoferrin from each other. Another method that has been proposed is to use silica particles coated with dextran to which carboxyl groups or sulfonic groups are introduced, and to elute lactoperoxidase and lactoferrin systematically by salt concentration gradient (Japanese Patent Laid-Open Publication No. 1-86839). This method, however, calls for further chromatography to separate lactoperoxidase and lactoferrin from each other completely.

Also known is the method of allowing milk to contact a polysaccharide affinity resin to which sulfonic groups are introduced, then washing the resin with aqueous solution of pH 5-9 containing 0.3 mole sodium chloride or with buffer solution, and eluting lactoferrin with 1.0 mole sodium chloride solution (Japanese Patent Laid-Open Publication No. 63-255300). This method, while providing lactoferrin in one chromatography, has a problem of somewhat lower purity and recovery of lactoferrin.

JP-A-63.255 299 und JP-A-63.255 300 disclose a method of separating lactoferrin from milk, comprising: contacting with a polysaccharide affinity resin, to which sulfonic groups are introduced, washing the resin an ionic strength of 0.3 and a pH of 5 to 9 and eluting lactoferrin at an ionic strength of 1.0. However, there is no disclosure in this prior art as regards elution conditions for lactoperoxidase.

The present invention aims at seperating and purifying lactoperoxidase and lactoperoxidase plus lactoferrin in a good yield and at high purity from solutions that contain: proteins capable of binding iron, by means of single chromatography.

### Detailed Description of Preferred Embodiments

The present invention relates to the method of separating, purifying and recovering lactoperoxidase or lactoperoxidase and lactoferrin, both proteins capable of binding iron, from solutions containing those proteins capable of binding iron, which comprises :
(a) contacting a solution that contains proteins capable of binding iron with a polysaccharide affinity resin, to which sulfonic groups are introduced, so that it adsorbs proteins capable of binding iron;
(b) washing the above resin with an aqueous solution or buffer solution of 0.2 or lower ionic strength and pH 5 or lower;
(c) eluting lactoperoxidase that has been adsorbed by the above resin with an aqueous solution or buffer solution of 0.2-0.7 ionic strength and pH 5 or lower;
(d) and, if wanted; eluting lactoferrin that has been adsorbed by the above resin with an aqueous solution or buffer solution of 0,5 or higher ionic strength and pH higher than 5.
(e) and recovering these lactoperoxidase and lactoferrin.

In the present invention, if preferred, the resin may be washed between step (a) and step (b) with aqueous solution or buffer solution of 0.1 or lower ionic strength and pH 5 or higher. The elution of lactoperoxidase in step (c) and the elution of lactoferrin in step (d) may be performed consecutively, or step (c) alone may be performed to elute and collect lactoperoxidase only.

In the present invention, the use of a resin that has specific affinity to lactoperoxidase and lactoferrin alone virtually eliminates all proteins except lactoperoxidase and lactoferrin, adsorbing only lactoperoxidase and lactoferrin that are then separated from each other by single chromatograpy.

Now on the market as an affinity resin that has specific affinity to lactoperoxidase and lactoferrin include heparin-Sepharose (by Pharmacia) and blue- Sepharose (by Pharmacia); or polysaccharide affinity resins including sultonated Cellulofine (by Chisso) in which sulfonic groups are introduced into cellulose, and sulfonic-group Chitopearl (by Fuji Spinning), that has sulfonic groups introduced into chitosan. However, heparin-Sepharose and blue-Sepharose cannot withstand high rate of flow and are too expensive for industrial use. Sulfonated Cellulofine and sulfonic- group Chitopearl, on the other hand, endure high rate of flow and are suitable for industrial use.

The inventors of the present invention found that, in order to curb adsorption of protein other than lactoperoxidase and lactoferrin as far as possible, and to improve adsorption of lactoperoxidase and lactoferrin, the amount of sulfonic group to be introduced to those polysaccharide affinity resins was important.

It was found necessary to adjust the amount of sulfonic group introduced to the resin to 10-50 µeq, preferably 27-40 µeq per one milliliter of resin. If the amount of sulfonic group introduced is 10 µeq or lower, adsorption of lactoperoxidase and lactoferrin will be less, thus lowering the yield of recovery. If the amount of sulfonic group introduced is 50 µeq or more, proteins other than lactoperoxidase and lactoferrin will also be adsorbed, resulting in lower purity. Resins, to which sulfonic group is introduced, could be Cellulofine (Chisso) or Chitopearl (Fuji Spinning ) among others, but the amount of recovered lactoperoxidase and lactoferrin is greatest when sulfonated Chitopearl is used.

The above amount of sulfonic group to be introduced to the resin has been determined by the following method.

5ml of the resin is filled into a column (Econo-column Chromatography column, 10 x 100mm, from Bio-rad), to which 100ml 1N HNO₃ is passed at a speed of 250ml per hour. The resin is then washed by passing water, purified with ion-exchange resin, at 250ml per hour until the washing water indicates neutral on Methyl Red. An appropriate amount of 1 mol NaCl is subsequently passed through the column, from which the first 50ml of the effluent solution is taken out and mixed well. Each 10ml of this solution is measured and poured into three conical flasks. Each is titrated with 0.01N NaOH, with phenolphthalein as an indicator. The amount of sulfonic group is obtained from the average of these three values

The above figure may vary somewhat if distilled, extra-purified water is used instead of water simply purified with ion-exchange resin: i.e. 10-80 µeq instead of 10-50 µeq. When this method is used, therefore, the amount of sulfonic group to be introduced to the resin must be adjusted to 10-80 µeq per 1ml resin, preferably 30-60 µeq per 1ml resin.

Next, lactoperoxidase and lactoferrin are separated from each other using eluants with adjusted ionic strength and pH. Since lactoperoxidase and lactoferrin are similar in molecular weights and isoelectric points, it is impossible to separate these two making use of ionic differences alone.

The resin that has adsorbed the proteins are first washed with aqueous solution or buffer solution of 0.2 or lower ionic strength and pH 5 or lower to eliminate impurities which is packed in the resin. Lactoperoxidase adsorbed by the resin may elute if ionic strength is higher than 0.2 or pH higher than 5, so it is desirable to do the washing with solution of 0.05-0.15 ionic strength and pH 2.5-4.5.

Prior to this washing, the resin that has adsorbed lactoperoxidase and lactoferrin may be pre-washed with aqueous solution or buffer solution of 0.1 or lower ionic strength and of pH 5 or higher. Cold or warm water may also be used for this preliminary washing. Thorough preliminary washing reduces the amount of aqueous solution or buffer solution, of 0.2 or lower ionic strength and of pH 5 or lower, needed in the subsequent washing, which amounts to substantial saving and is advantageous when production costs are taken into account.

The kind of buffer solution used for the subsequent washing is not particularly restricted, and any buffer solution will do if it has buffer capacity of 0.2 or weaker ionic strength and of pH 5 or lower. Examples of buffer solutions include an organic acid buffer solution such as acetate buffer and citrate buffer, and glycine buffer, and β,β'-dimethylglutarate buffer. Phosphate buffer may also be used, but its buffer capacity decreases at pH 5 or lower.

Next, lactoperoxidase and lactoferrin that have been adsorbed by the resin are eluted separately.

The present invention enables efficient separation and elution with single chromatography, since the resin is first washed with cleaning solution of specific ionic strength and pH, and then eluted with aqueous solution or buffer solution of specific ionic strength and pH as eluant.

To elute lactoperoxidase that is adsorbed by the resin, aqueous solution or buffer solution of 0.2-0.7 ionic strength and pH 5 or lower, preferably that of 0.4-0.6 ionic strength and pH 2.5-4.5, is used. Lactoperoxidase will not be eluted or eluted only in very small amount, if ionic strength is 0.2 or lower.

If the ionic strength is 0.7 or higher, eluted lactoperoxidase will be less pure because lactoferrin will also be eluted at the same time, even if pH is 5 or lower, which will subsequently result in smaller amount of lactoferrin recovery. If pH is 5 or higher, on the other hand, purity of lactoperoxidase will also suffer because a small amount of lactoferrin is eluted at the same time, even if the ionic strength is 0.2 or lower.

The kind of buffer solution used here is not particularly restricted, either, allowing the use of an organic acid buffer such as acetate buffer and citrate buffer glycine buffer, and β,β'-dimethylglutarate buffer. To increase ionic strength, adding some sodium chloride is sufficient. Purity of lactoperoxidase thus obtained is at least 50%, and lactoperoxidase of 80% purity or higher can usually be obtained with ease.

Following the elution of lactoperoxidase that is adsorbed by the resin, lactoferrin is then eluted using aqueous solution or buffer solution of 0.5 or higher ionic strength and of a pH higher than 5, preferably 0.7-2.0 ionic strength and pH 6-8. Amount of lactoferrin eluted will be small if ionic strength is 0.5 or lower, or if pH is 5 or lower.

The kind of aqueous solution or buffer solution used here is not particularly limited, and examples include: aqueous solution made by dissolving sodium chloride in water to adjust ionic strength to 0.5 or higher; organic acid buffer solution such as citrate buffer, acetate buffer, malate buffer ; or phosphate buffer, imidazole buffer, sodium bicarbonate buffer, borate buffer, Tris buffer. Purity of lactoferrin thus obtained is at least 70%, with lactoferrin of 80% purity or higher is usually obtained.

These processes of eluting lactoperoxidase and lactoferrin may be performed independently as needed, which will provide highly pure lactoperoxidase.

Finally, washing the resin with cold or warm water regenerates it, allowing repeated use for purifying lactoperoxidase and lactoferrin.

The kind of reaction unit used for the adsorption and elution processes is not particularly limited to specific types, and any of the pack-layer type, fluid-layer type or stirred-layer type, may be used, although rotary-type column seem most appropriate as it won't trigger channeling even at high rate of flow, and will not compress the resin.

Skimmed milk and whey are examples of solutions that contain proteins capable of binding iron, which is the starting material of the process. Those pasteurized under heat can be used, but unheated solution is preferable, because heating will not only cause lactoperoxidase to lose its activity and cause lactoferrin to become denatured, it will also lower their purity as well as the yield of recovery due to interaction with other proteins.

Lactoperoxidase and lactoferrin obtained by the process described above are desalinated by either ultrafiltration, electrodialysis, or other method, and then dried in the usual manner, after which they are ready for use in for example pharmaceuticals and food products.

### Effect of the Invention

Separating and recovering lactoperoxidase and lactoferrin in accordance with the present invention eliminates the need, as in conventional methods, for further chromatography to separate lactoperoxidase and lactoterrin from each other and to purify them, thus simplifying the entire process. Specillcally, the present invention enables recovery of lactoperoxidase and lactoferrin of 80% or higher purity with one single chromatography. An addition of ultrafiltration process will eliminate the slight amount of low-molecular-weight impurities, ultimately rendering lactoperoxidase and lactoferrin of 90% or greater purity.

Simplification of the process, high purity of the product and high yield of recovery make it possible not only to lower production costs but also to utilize the proteins in food products and pharmaceuticals for preventing infection, food products and pharmaceuticals for preventing or treating anemia.

The following are Examples carried out to demonstrate the present invention.

### Example 1

One milliliter of sulfonated Chitopearl (by Fuji Spinning), to which 29.4 µeq sulfonic group per milliliter resin had been introduced, was packed into a column of 1.0 centimeters by 4.0 centimeters, through which 100 milliliters of raw skimmed milk was passed at a flow rate of 240 milliliters per hour. The skimmed milk contained 3 milligrams lactoperoxidase and 10 milligrams lactoferrin. Then, the resin was first washed with 100 milliliters of deionized water, and was subsequently washed with 50 milliliters aqueous solution that contained 0.1 mole sodium chloride solution (I=0.1) and had been adjusted to pH 3.5 with diluted hydrochloric acid. Next, lactoperoxidase was collected by passing 100 milliliters of 0.01 mole phthalate buffer (pH 4.5) containing 0.4 mole sodium chloride (I=0.4). Furthermore, lactoferrin was collected by passing 100 milliliters of 0.01 mole phosphate buffer (pH 7.6) that contains 1.0 mole sodium chloride (I=1.0). Finally, 300 milliliters of deionized water was passed through the column to regenerate the resin.

Thus, 2.4 milligrams lactoperoxidase and 8.2 milligrams lactoferrin were collected. Purity of lactoperoxidase and lactoferrin thus obtained were 82% and 85%, respectively.

### Example 2

Sulfonated Celluloline, 10 milliliters, to which 33 µeq sulfonic group per milliliter resin had been introduced, was placed in a 500 milliliter-capacity beaker, and, together with 200 milliliters raw skimmed milk, was stirred for 40 minutes at room temperature. After decanting away the skimmed milk, the resin was washed with 0.005 mole acetate buffer (pH 6.0) with 0.05 mole sodium chloride (I=0.05), after which it was further washed with 0.005 mole acetate buffer (pH 4.0) with 0.15 mole sodium chloride (I=0.15) added. Following the decantation of this buffer, another 50 millimeters of 0.005 mole acetate buffer (pH 4.0) with 0.5 mole sodium chloride (I=0.5) was poured over the resin and was stirred together for 10 minutes, after which 40 milliliters supernatant was obtained by decantation, from which lactoperoxidase was recovered.

This resin was subsequently stirred for 10 minutes together with 50 milliliters aqueous solution that contain 0.8 mole sodium chloride (I=0.8) and had been adjusted to pH 8.0 with diluted sodium hydroxide, after which supernatant was obtained by decantation. Repeating this process twice rendered a total 75 milliliters of supernatant, from which lactoferrin was obtained. Finally, the resin was stirred with 100 milliliters warm water (40 degrees Celsius) for ten minutes. This operation was repeated five times to regenerate the resin.

Thus recovered were 3.8 milligrams of lactoperoxidase with 83% purity and 12.5 milligrams of lactoferrin with 85% purity.

### Example 3

1.7 liters of sulfonated Chitopearl (by Fuji Spinning), to which 38.9 µeq sulfonic group per milliliter resin had been introduced, was packed into a rotary adsorption-desorption unit of 2.3-liter capacity.

Then, 100 liters of unheated cheese whey, which had been passed through a clarifier to eliminate curd, was circulated through the above unit for one hour at a flow rate of 200 liters per hour. The cheese whey contained about 4 grams lactoperoxidase and around 8 grams lactoferrin. After completing the circulation, 40 liters warm water was passed through the unit, and the resin was further washed with 20 liters of 0.005 mole citrate buffer (pH 4.0) containing 0.1 mole sodium chloride (I=0.1). Lactoperoxidase was then eluted by passing through the unit 20 liters of 0.005 mole citrate buffer (pH 4.0) containing 0.5 mole sodium chloride (I=0.5). Purity of lactoperoxidase at this step was 85%. Lactoferrin was subsequently eluted by passing through the unit 20 liters aqueous solution, prepared by adding sodium bicarbonate to 1.0 mole sodium chloride solution (I=1.0) to adjust pH to 6.8. Purity of lactoferrin at this step was 85%. Lastly, 60 liters of warm water (40 degrees Celsius) was passed through to regenerate the resin.

Solution of lactoperoxidase and that of lactoferrin were then ultrafiltrated with an ultrafiltration unit (Nitto Denko RUW-4) equipped with ultrafiltration membrane of molecular weight cut-off 50 kDa (Nitto Denko NPU 3150 S2), and were further desalinating by diafiltration using the same unit. After desalinating each was freeze-dried to obtain 3.8 grams of lactoperoxidase and 7.4 grams of lactoferrin. Final purity of lactoperoxidase stood at 92%, and that of lactoferrin, also at 92%.

### Example 4

As in Example 3, 100 liters cheese whey was circulated through the same unit, and the sulfonated Chitopearl was washed first with warm water and then with 0.005 mole citrate buffer containing 0.1 mole sodium chloride (I=0.1), after which its pH was adjusted to 7.5 with potassium carbonate, followed by passing of 20 liters aqueous solution containing 0.8 mole sodium chloride (I=0.8). This eluate was ultrafiltered as in Example 3 to concentrate and was next desalinated by diafiltration. The freeze-dried mass contained 3.5 grams of lactoperoxidase and 7.3 grams of lactoferrin.

## Claims

1. A method of separating, purifying and recovering proteins capable of binding iron, namely lactoperoxidase or lactoperoxidase and lactoferrin, from solutions containing these proteins capable of binding iron, ***characterized*** by that the processes (a) through (d), description of which follows, are either performed consecutively, or that the processes (a) and (b) are performed, followed by implementation of only (c); wherein
(a) contacting a solution containing the proteins capable of binding iron with a polysaccharide affinity resin, to which sulfonic groups are introduced, so that the resin adsorbs the proteins capable of binding iron, (b) after which this resin is washed with an aqueous solution or buffer solution of 0.2 or lower ionic strength and of pH 5 or below,
(c) followed by elution of lactoperoxidase that has been adsorbed by the above resin, using an aqueous solution or buffer solution of 0.2-0.7 ionic strength and of pH 5 or below,
(d) and, if wanted, followed by elution of lactoferrin that has been adsorbed by the above resin, using an aqueous solution or buffer solution of 0.5 or higher ionic strength and of a pH higher than 5.

2. A method according to claim 1 ***characterized*** in that after step (a) and in advance of step (b) the resin is washed with an aqueous solution or buffer solution of 0.1 or lower ionic strength and of pH 5 or higher.

3. A method according to claim 1 or claim 2, wherein the polysaccharide affinity resin, to which sulfonic groups are introduced, is polysaccharide affinity resin to which 10-50 µeq of sulfonic group per 1 milliliter of resin is introduced.

4. A method according to any of claims 1 through 3, wherein the polysaccharide affinity resin, to which sulfonic groups are introduced, is chitosan.

5. A method according to any of claims 1 through 4, wherein the solution containing the proteins capable of binding iron is either skimmed milk or whey.

## Patentansprüche

1. Verfahren zur Abtrennung, Reinigung und Gewinnung von Proteinen, die Eisen binden können, und zwar Lactoperoxidase oder Lactoperoxidase und Lactoferrin, von Lösungen, die diese Proteine enthalten, die Eisen binden können, dadurch **gekennzeich****net**, daß man die folgenden Stufen (a) bis (d) entweder nacheinander durchführt oder daß man die Stufen (a) und (b) durchführt, wonach man nur Stufe (c) durchführt, wobei man
(a) eine Lösung, die die Proteine enthält, die Eisen binden können, mit einem Harz mit Polysaccharidaffinität kontaktiert, das mit Sulfongruppen versehen ist, so daß das Harz die Proteine adsorbiert, die Eisen binden können,
(b) danach das Harz mit einer wässerigen Lösung oder einer Pufferlösung einer Ionenstärke von 0,2 oder weniger und einem pH von 5 oder weniger wäscht,
(c) danach Lactoperoxidase eluiert, die vom Harz adsorbiert wurde, wobei man eine wässerige Lösung oder eine Pufferlösung einer Ionenstärke von 0,2 bis 0,7 und mit einem pH von 5 oder weniger verwendet,
(d) und gegebenenfalls danach Lactoferrin eluiert, das vom genannten Harz adsorbiert wurde, wobei man eine wäßrige Lösung oder eine Pufferlösung einer Ionenstärke von 0,5 oder mehr und mit einem pH von mehr als 5 verwendet.

2. Verfahren nach Anspruch 1, dadurch **gekennzeichnet**, daß man nach Stufe (a) und vor Stufe (b) das Harz mit einer wäßrigen Lösung oder einer Pufferlösung einer Ionenstärke von 0,1 oder weniger und mit einem pH von 5 oder mehr wäscht.

3. Verfahren nach Anspruch 1 oder 2, bei dem das Harz mit Polysaccaridaffinität, das mit Sulfongruppen versehen ist, ein Harz mit Polysaccharidaffinität ist, das mit 10 bis 50 µeq Sulfongruppen pro 1 ml Harz versehen ist.

4. Verfahren nach einem der Ansprüche 1 bis 3, bei dem das Harz mit Polysaccharidaffinität, das mit Sulfongruppen versehen ist, Chitosan ist.

5. Verfahren nach einem der Ansprüche 1 bis 4, bei dem die Lösung, die die Proteine enthält, die Eisen binden können, Magermilch oder Molke ist.

## Revendications

1. Procédé de séparation, purification et récupération de protéines capables de fixer du fer, à savoir la lactoperoxydase ou la lactoperoxydase et la lactoferrine, à partir de solutions contenant ces protéines capables de fixer du fer, caractérisé en ce que soit l'on effectue consécutivement les procédés (a) à (d), dont la description suit, soit l'on effectue les procédés (a) et (b) suivis par la réalisation de (c) seulement ; avec
(a) la mise en contact d'une solution contenant les protéines capables de fixer du fer avec une résine d'affinité polysaccharide, dans laquelle on introduit des groupes sulfoniques, de telle sorte que la résine adsorbe les protéines capables de fixer du fer,
(b) puis le lavage de cette résine avec une solution aqueuse ou une solution tampon de force ionique valant 0,2 ou moins et de pH valant 5 ou au-dessous,
(c) suivi de l'élution de la lactoperoxydase qui a été adsorbée par la résine ci-dessus ,avec une solution aqueuse ou une solution tampon de force ionique de 0,2 - 0,7 et de pH valant 5 ou moins,
(d) et, si on le souhaite, suivie de l'élution de la lactoferrine qui a été adsorbée par la résine ci-dessus,à l'aide d'une solution aqueuse ou d'une solution tampon de force ionique valant 0,5 ou plus et de pH supérieur à 5.

2. Procédé selon la revendication 1, caractérisé en ce que après l'étape (a) et avant l'étape (b), on lave la résine avec une solution aqueuse ou une solution tampon d'une force ionique valant 0,1 ou moins et d'un pH de 5 ou plus.

3. Procédé selon la revendication 1 ou 2, dans lequel la résine d'affinité polysaccharide, dans laquelle on introduit des groupes sulfoniques,est une résine d'affinitié polysaccharide dans laquelle on introduit 10 - 50 µeq de groupes sulfoniques pour 1 millilitre de résine.

4. Procédé selon l'une quelconque des revendications 1 à 3, dans lequel la résine d'affinité polysaccharide,dans laquelle on introduit des groupes sulfoniques, est le chitosane.

5. Procédé selon l'une quelconque des revendications 1 à 4, dans lequel la solution contenant les protéines capables de fixer du fer est soit du lait écrémé soit du petit lait.
